Europäisches Patentamt

European Patent Office

Office européen des brevets

(11) Veröffentlichungsnummer: **0 069 850**
A2

# (12) EUROPÄISCHE PATENTANMELDUNG

(21) Anmeldenummer: 82104619.0

(22) Anmeldetag: 27.05.82

(51) Int. Cl.³: **A 61 B 7/02**

(30) Priorität: 11.07.81 DE 3127526

(43) Veröffentlichungstag der Anmeldung:
19.01.83 Patentblatt 83/3

(84) Benannte Vertragsstaaten:
GB NL

(71) Anmelder: Kirchner & Wilhelm
Heusteigstrasse 70 A
D-7000 Stuttgart-1(DE)

(72) Erfinder: Bühler, Herbert
Taubenheimstrasse 106
D-7000 Stuttgart 50(DE)

(74) Vertreter: Patentanwälte Dipl.-Ing. B. Schmid Dr.-Ing.
G.A. Birn Dipl.-Ing. H. Quarder
Falbenhennenstrasse 17
D-7000 Stuttgart 1(DE)

(54) Stethoskop.

(57) Um das reibende Drehen der Oliven od. dgl. eines Stethoskops im Ohr des Arztes bei einer Lageveränderung seines Kopfes zu vermeiden, werden die Ohrstücke (3) um ihre Längsachse (4) drehbar am Ohrbügel (1) gehalten. Die Ohrstücke (3) sind zweiteilig aus gebildet, wobei das äußere Teil als Olive (6) aus gebildet ist und leicht abgezogen werden kann, während das rohrförmige Innenteil (5) drehbar, aber nicht abnehmbar mit dem Ohrbügelarm (2) verbunden ist.

EP 0 069 850 A2

-1-

Kirchner & Wilhelm

Heusteigstr. 70 A

7000 Stuttgart 1

Stethoskop

Die Erfindung bezieht sich auf ein Stethoskop mit an den beiden Enden seines Ohrbügels angebrachten Ohrstücken. Stethoskope sind in den Heilberufen schon lange bekannt und praktisch unentbehrliche Hilfsgeräte. Beim Abhören eines Patienten steckt sich der Arzt od. dgl. die Ohrstücke in seine Ohren, und der Ohrbügel hängt dabei nach unten. Wenn sich der Arzt aufrichtet oder nach vorne beugt, aber auch bei einer Lageänderung des Patienten, kommt es zu einer Drehung des Ohrbügels mit den Ohrstücken als Drehpunkt. Weil die Ohrstücke fest mit dem Ohrbügel verbunden sind, bewirkt dieses Drehen des Ohrbügels ein Reiben

– 2 –

der Ohrstücke auf der Haut am Gehörganganfang, und dies wird
zumindest als unangenehm, gelegentlich aber auch als Schmerz
empfunden.

Die Aufgabe der Erfindung besteht infolgedessen darin, ein Stethoskop der eingangs genannten Art zu schaffen, welches das unangenehme Reiben der Ohrstücke im Ohr des Arztes od. dgl. vermeidet.

Zur Lösung dieser Aufgabe wird erfindungsgemäß vorgeschlagen,
daß bei einem Stethoskop gemäß dem Oberbegriff des Anspruchs 1
die Ohrstücke um ihre Längsachse drehbar am Ohrbügel gehalten
sind. Hierbei ist zu bedenken, daß die freien Enden des Ohrbügels in Gebrauchslage etwa gegeneinander weisen und sie bei
normaler Kopflage des Arztes eine horizontale Stellung einnehmen. Aufgrund der nunmehr drehbaren Verbindung der Ohrstücke
mit dem Ohrbügel kann sich letzterer bei Veränderung der Kopfneigung des Arztes jeweils auspendeln, jedoch behalten dabei
die Ohrstücke ihre Relativlage zum Ohr bei, so daß die unangenehme Reibung am Gehörganganfang unterbleibt.

Ein weiterer Vorteil dieses Stethoskops liegt darin, daß nach
dem Aufsetzen des Ohrbügels die bestmögliche Stellung der Ohrstücke im Ohr gesucht werden kann und diese Stellung unabhängig
von einer späteren Drehung des Ohrbügels um die durch die beiden Ohrstücke gebildete Drehachse beibehalten wird. Dadurch kommt es nicht zuu einem veränderten, insbesondere schlechteren Hören während der Untersuchung, das bislang durch die Bewegung von Arzt und/oder Patient nahezu unvermeidlich war. Zum angenehmeren Tragen kommt somit auch noch das bessere Hören hinzu.

Eine Weiterbildung der Erfindung sieht vor, daß das Ohrstück zweiteilig ausgebildet ist und aus einem rohrförmigen Teil sowie einer klemmend daran gehaltenen. sogenannten Olive besteht. Letztere ist in bekannter Weise aus Gummi. Kunststoff od. dgl. gefertigt, während man das Innenteil zweckmäßigerweise aus Metall herstellt. Die Olive wird auf das Innenteil lediglich aufgesteckt, und sie hält daran aufgrund der Eigenspannung des Materials bei entsprechender Dimensionierung der Durchmesser. Somit können die Oliven rasch ausgewechselt und gereinigt oder ggf. durch andere ersetzt werden.

Eine Weiterbildung der Erfindung sieht vor, daß jedes freie Ende des Rohrbügels absatzartig im Querschnitt reduziert und das rohrförmige Innenteil mit Spiel auf das dünnere Rohrbügelende aufgeschoben ist, wobei ein Halteglied des Rohrbügels das Innenteil gegen Herausfallen sichert. Das Halteglied ist so auszubilden und anzuordnen, daß es einerseits das Drehen des Ohrstücks nicht behindert und andererseits auch keinen nachteiligen Einfluß auf das Hörergebnis nimmt. Eine in dieser Hinsicht besonders vorteilhafte Ausgestaltung der Erfindung besteht darin, daß das Halteglied des Ohrbügels durh dessen

aufgedorntes, freies Ende gebildet ist. Man steckt also zunächst das rohrförmige Innenteil auf das absatzartig reduzierte freie Ohrbügelende auf wobei die beiden Längen in etwa gleich sind. Nachfolgend dornt man das freie Rohrbügelende etwas auf, wodurch ein Außenbund entsteht. der das Herausziehen des rohrförmigen Innenteils verhindert. andererseits aber dessen Drehung ohne weiteres zuläßt. Das axiale Spiel wird vorzugsweise etwas größer gewählt als das radiale, welches man im Hinblick auf gute akustische Verhältnisse möglichst klein wählt.

Eine weitere Ausbildung der Erfindung besteht darin. daß sich das aufgedornte freie Ende des Ohrbügels in einer konischen Erweiterung des äußeren Bohrungsendes des Innenteils befindet. Dadurch läßt sich einerseits beim Aufdornen die Aufweitung leicht begrenzen. und andererseits kann man einen bündigen oder zumindest weitgehend bündigen Abschluß am Aufsteckende für die Olive erzielen.

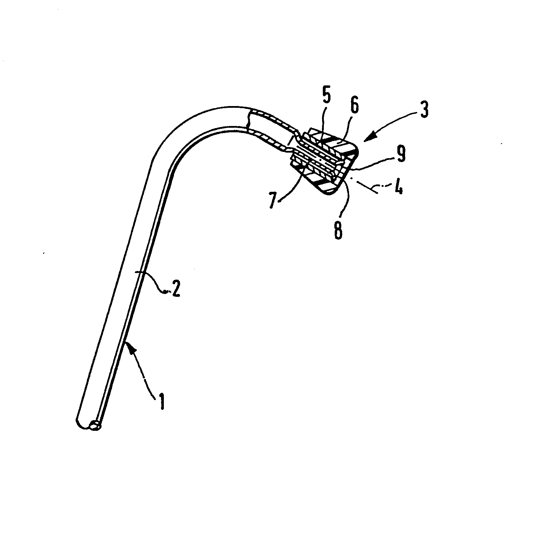

In der Zeichnung ist ein Ausführungsbeispiel der Erfindung anhand eines Längsschnitts durch das eine Ende eines Stethoskop-Ohrbügels dargestellt.

Das Stethoskop besitzt einen Ohrbügel 1. dessen linker Ohrbügelarm 2 teilweise gezeichnet ist. Das freie Ohrbügelende ist in bekannter Weise bogenförmig ausgebildet, und der Ohrbügel selbst besteht wenigstens teilweise aus vorzugsweise ver-

— 5 —

chromten Rohren. Über diese wird der Schall, der beim Abhören
des Patienten aufgenommen wird, zum Ohr des Arztes od. dgl.
geleitet.

Gemäß der Erfindung sind die beiden Ohrstücke 3 um ihre Längsachse 4 drehbar am Ohrbügel bzw. ihrem Ohrbügelarm gehalten. Zu
diesem Zweck sind sie zweiteilig ausgebildet, und sie bestehen
beim Ausführungsbeispiel aus einem rohrförmigen Innenteil 5 und
einer aufgesteckten, klemmend daran gehaltenen Olive 6. Letztere besteht in bekannter und vorteilhafter Weise aus Gummi,
Kunststoff odd. dgl. Das rohrförmige Innenteil stellt man am
besten aus Metall her, wobei es, wie bei medizinischen Geräten
üblich, verchromt sein kann.

Die Ohrstücke 3 sind als Ganzes drehbar und im Rahmen der Toleranzen geringfügig in axialer Richtung verschiebbar. Abnehmbar
ist, wie gesagt, lediglich die Olive 6, nicht jedoch das rohrförmige Innenteil 5. Man erreicht das dadurch, daß man das
freie Ende 7 des Ohrbügels absatzartig im Querschnitt reduziert
und das rohrförmige Innenteil 5 mit Spiel auf das dünnere Ohrbügelende aufschiebt. Der Absatz bildet einen Verschiebeanschlag.

In der Gegenrichtung erhält man einen Verschiebeanschlag durch
Aufdornen des freien Endes des Ohrbügelarms 2 bzw. seines im
Querschnitt reduzierten Endstücks, wodurch ein Außenbund 9

— 6 —

entsteht. Demzufolge kann man das freie Ohrbügelende erst nach dem Aufschieben des rohrförmigen Innenteils aufdornen. Damit die beiden Teile in etwa bündig abschließen, bringt man am äußeren Ende der Bohrung des rohrförmigen Innenteils eine Ansenkung bzw. konische Erweiterung 8 an. Abschließend steckt man dann die Olive 6 auf, welche das rohrförmige Innenteil 5 und das aufgedornte Ende des Ohrbügelarms 2 etwas überragt, so daß diese freien Enden mit dem Ohr des Arztes nicht in Berührung kommen können.

- 1 -

14 248

A n s p r ü c h e

1. Stethoskop mit an den beiden Enden seines Ohrbügels (1) angebrachten Ohrstücken (3) dadurch gekennzeichnet daß die Ohrstücke (3) um ihre Längsachse (4) drehbar am Ohrbügel (1) gehalten sind.

2. Stethoskop nach Anspruch 1 dadurch gekennzeichnet, daß jedes Ohrstück (3) zweiteilig ausgebildet ist und aus einem rohrförmigen Innenteil (5) sowie einer klemmend daran gehaltenen sogenannten Olive (6) besteht.

3. Stethoskop nach Anspruch 2 dadurch gekennzeichnet daß jedes freie Ende des Ohrbügels (1) absatzartig im Querschnitt reduziert und das rohrförmige Innenteil (5) mit Spiel auf das dünnere Ohrbügelende aufgeschoben ist, wobei ein Halteglied (9) des Ohrbügels (1) das rohrförmige Innenteil (5) gegen Herausfallen sichert.

4. Stethoskop nach Anspruch 3. dadurch gekennzeichnet, daß das Halteglied (9) des Ohrbügels (1) durch dessen aufgedorntes freies Ende gebildet ist.

5. Stethoskop nach Anspruch 4 dadurch gekennzeichnet, daß sich das aufgedornte freie Ende (9) des Ohrbügels (1) in einer konischen Erweiterung (8) des äußeren Bohrungsendes des rohrförmigen Innenteils (5) befindet.